(19) Europäisches Patentamt

European Patent Office

Office européen des brevets

(11) Veröffentlichungsnummer: **0 502 298 A2**

# EUROPÄISCHE PATENTANMELDUNG

(21) Anmeldenummer: **92101070.8**

(22) Anmeldetag: **23.01.92**

(51) Int. Cl.5: **C07H 11/04**, C07H 19/207

(30) Priorität: **31.01.91 DE 4102817**

(43) Veröffentlichungstag der Anmeldung:
**09.09.92 Patentblatt 92/37**

(84) Benannte Vertragsstaaten:
**AT BE CH DE DK ES FR GB GR IT LI LU NL SE**

(71) Anmelder: **MERCK PATENT GESELLSCHAFT MIT BESCHRÄNKTER HAFTUNG**
**Frankfurter Strasse 250**
**W-6100 Darmstadt(DE)**

(72) Erfinder: **Schmidt, Richard R.,Prof.Dr.**
**Grossherzog-Friedrich-Strasse 11**
**W-7750 Konstanz 16(DE)**
Erfinder: **Jung, Karl-Heinz, Dr.**
**Espenstrasse 11**
**W-7750 Konstanz 16(DE)**
Erfinder: **Spangehl, Barbara,Dr.**
**Am Stutenanger 7a**
**W-8042 Oberschleissheim(DE)**
Erfinder: **Kinzy, Willy, Dr.**
**Schlossstrasse 19 b**
**W-7821 Inzingen(DE)**
Erfinder: **Hemberger, Jürgen, Dr.**
**Konradstrasse 7 a**
**W-8750 Aschaffenburg(DE)**

(54) Verfahren zur stereoselekfiven Herstellung von beta-Fucopyranosylphosphaten und sehr reiner GDP-Fucose.

(57) Die Erfindung betrifft ein Verfahren zur stereoselektiven Herstellung von $\beta$-L-Fucopyranosylphosphaten über die Trichloracetimidate der geschützten L-Fucose und die Synthese und Aufreinigung von sehr reiner GDP-Fucose aus den $\beta$-L-Fucopyranosyl-phosphaten.

EP 0 502 298 A2

Die Erfindung betrifft ein Verfahren zur stereoselektiven Herstellung von β-Fucopyranosyl-phosphaten und von sehr reiner Guanosin-diphosphat-fucose (GDP-Fucose), die aus diesen hergestellt werden kann.

L-Fucose ist bekannt als Bestandteil von Oligosacchariden der Milch sowie von Blutgruppensubstanzen (z.B.: Hakomori, Progr. Biochem. Pharmacol. 10 (1975), 167). Die Desoxyhexose ist ferner beteiligt beim Aufbau von Lipopolysacchariden von Bakterien und Glycosphingolipiden von Säugern (z.B.: Flowers, Adv. Carbohydr. Chem. 39 (1981), 279). Fucosylierte Glycolipide spielen eine wichtige Rolle bei der Regulation des Zellwachstums und der Zelldifferenzierung. Von besonderem Interesse ist aber, daß fucosylierte Glycolipide Bestandteile von Oberflächenstrukturen von Tumorzellen sind und antigene Eigenschaften entfalten (z.B.: Hakomori, Ann. Rev. Biochem. 50 (1981), 733). Sie erlangen somit Bedeutung für die Tumortherapie und Tumordiagnostik. Die Biosynthese von Fucose enthaltenden Oligosacchariden und Glycolipiden bedarf unter anderem bestimmter Enzyme, den Fucosyltransferasen. Substrat für diese Fucosyltransferasen ist Guanosindiphosphat-fucose (GDP-Fucose).

Zur Untersuchung und Erzeugung von tumor-assoziierten Antigenen auf Basis von fucosylierten Glycolipiden ist es daher erforderlich, größere Mengen an sehr reiner GDP-Fucose zur Verfügung zu haben. Trotz des seit längerer Zeit bestehenden Bedarfs sind nur eine chemische (Nunez et al., Can. J. Chem. 59 (1981), 2086) und mehrere enzymatische Synthesen (z.B.: Yamamoto et al., Agric. Biol. Chem. 48 (1984), 823) von GDP-Fucose bekannt geworden. Die enzymatischen Synthesen, die beispielsweise über Fucose-1-phosphat oder über GDP-Mannose ablaufen, haben allerdings den Nachteil, daß sie nur geringe Mengen GDP-Fucose liefern.

Die Schlüsselverbindung in der chemischen Synthese von GDP-Fucose ist zweifelsohne Fucose-1-phosphat, dessen Bereitstellung in ausreichender Menge die limitierende Größe innerhalb der Gesamtsynthese ist. Fucose-1-phosphat tritt in zwei unterschiedlichen Konfigurationen auf, und zwar in Form des α-Fucopyranosyl-phosphats, das sich relativ einfach gemäß der Chlorphosphoamidat-Methode (z.B.: Westerduin et al., Tetrahedron Leit. 27 (1986), 1211) herstellen laßt, und in Form des β-Fucopyranosyl-phosphats. Zur Synthese von GDP-Fucose eignet sich ausschließlich das β-konfigurierte Phosphat. Die bekannten chemischen Synthesen von β-Fucopyranosyl-phosphat (Nunez et al., l. c.; Tsai et al., Carbohydr. Res. 64 (1978), 297) zeichnen sich jedoch durch eine niedrige Stereoselektivität und/oder niedrige Ausbeute aus und sind zudem teilweise aufwendig und kompliziert, sodaß sie sich bestenfalls für eine Herstellung im Labormaßstab eignen. Die Schwierigkeit in der bisherigen Herstellung von β-Fucopyranosyl-phosphat ist wohl in dessen ausgeprägter Labilität, insbesondere gegenüber Säuren, zu suchen.

Die bekannten Verfahren zur Herstellung von GDP-Fucose aus β-Fucopyranosyl-phosphat (Nunez et al., l. c.) weist weiterhin den Nachteil auf, daß die Aufreinigung des Endproduktes lediglich mittels eines Ionenaustauschers durchgeführt wird, so daß wohl für viele biochemische Zwecke möglicherweise eine weitere Aufreinigung zumindest aber eine Entsalzung des Produktes notwendig wird. Dies ist aber wieder mit Ausbeuteverlusten verbunden.

Es bestand somit die Aufgabe, ein neues Verfahren zur Herstellung von β-Fucopyranosyl-phosphat und daraus hergestellte GDP-Fucose zu entwickeln, welches sich durch hohe Stereoselektivität, gute Ausbeuten, hohe Reinheit des Endproduktes und Einfachheit des Versuchsablaufs auszeichnet.

Es wurde nun gefunden, daß man β-Fucopyranosyl-phosphate aus den α-Trichloracetimidaten von geschützter L-Fucose durch einfache Umsetzung mit sehr reinen organischen Phosphaten wie z.B. Dibenzyl- oder Diphenylphosphat in hohen Ausbeuten herstellen kann.

Es wurde auch gefunden, daß man die hierfür benötigten α-Trichloracetimidate leicht durch Umsetzung von L-Fucose bzw. ihren an den Positionen 2, 3 und 4 geschützten Formen stereoselektiv mit Trichloracetonitril unter Verwendung von vorzugsweise Natriumhydrid als Base in hohen Ausbeuten herstellen kann.

Letztlich wurde gefunden, daß die Bereitstellung von GDP-Fucose durch Umsetzung der erfindungsgemäß hergestellten β-Fucopyranosyl-phosphate mit aktiviertem GMP in einem weiteren erfindungsgemäßen Verfahrensschritt besonders vorteilhaft abläuft, wenn die β-Fucopyranosyl-phosphate in ein leicht lösliches Salz, insbesondere das Tri-n-octylammonium-Salz, überführt werden, wobei die hohe Reinheit der gebildeten GDP-Fucose durch Aufreinigung mittels HPLC unter Verwendung eines leicht verdampfbaren Eluenten erzielt und das Verfahren überdies vereinfacht werden kann.

Gegenstand der Erfindung ist somit ein Verfahren zur selektiven Herstellung von β-Fucopyranosyl-phosphaten, dadurch gekennzeichnet, daß man an den Positionen 2, 3 und 4 geschützte L-Fucose in das entsprechende O-(α-L-Fucopyranosyl)-trichloracetimidat überführt, dieses mit einem absolut säurefreiem organischem Phosphat der Formel $PO(OH)(OR)_2$, worin R $C_1$-$C_4$ - Alkyl, Benzyl oder Phenyl bedeutet, umsetzt, den Rest R der Phosphatgruppe und die Schutzgruppen am Fucopyranosylring abspaltet und das freie $\beta$-L-Fucopyranosyl-phosphat als Salz isoliert.

Gegenstand der Erfindung ist ferner ein Verfahren zur stereoselektiven Herstellung von sehr reiner GDP-Fucose, dadurch gekennzeichnet, daß man $\beta$-L-Fucopyranosyl-phosphats in ein leicht lösliches Salz überführt, dieses mit aktiviertem GMP umsetzt und die gebildete GDP-Fucose mittels präparativer HPLC mit einem leicht verdampfbaren Puffersystem als Eluent aufreinigt.

Gegenstand der Erfindung ist insbesondere ein entsprechendes Verfahren, das dadurch gekennzeichnet ist, daß das eingesetzte $\beta$-Fucopyranosyl-phosphat nach einem der beschriebenen Verfahren hergestellt wird.

Das erfindungsgemäße Verfahren zeichnet sich auch dadurch aus, daß die Bereitstellung von insbesondere Tri-O-acetyl-L-fucose aus der tetraacetylierten Verbindung in einer Einstufen-Reaktion, vorzugsweise mit Hydrazinacetat, durchgeführt werden kann, was zur Vereinfachung der Gesamtsynthese beiträgt.

Gegenstand der Erfindung ist also auch ein entsprechendes Verfahren, das dadurch gekennzeichnet ist, daß man 2,3,4-Tri-O-acetyl-L-fucose aus der tetraacetylierten L-Fucose in einer Einstufen-Reaktion herstellt.

Verwendet man bei der Umsetzung von 2,3,4-geschützter L-Fucose mit Trichloracetonitril andere Basentypen, entstehen unterschiedliche Gemische aus $\alpha$- und $\beta$-Fucopyranosyltrichloracetimidaten, die für den weiteren Einsatz erst getrennt werden müssen. Bei Einsatz von Natriumhydrid und analogen Basen wird ausschließlich das entsprechende $\alpha$-Trichloracetimidat gebildet. Die Ausbeuten von $\alpha$-Imidaten bei Einsatz von Natriumhydrid, bezogen beispielsweise auf die Tri-O-acetyl-L-fucose, betragen durchschnittlich 70 bis 80 %.

Der Reinheitsgrad, insbesondere der Restgehalt von Säuren, der zur Verwendung kommenden Phosphate vom Typ $P(OH)(OR)_2$ bei der Reaktion mit den $\alpha$-Trichloracetimidaten der geschützten L-Fucose zu den Fucopyranosyl-phosphaten bestimmt den stereoselektiven Verlauf in bezug auf die entstehenden Anomeren. Die $\alpha$-Fucopyranosyl-phosphate, welche für die Weiterverarbeitung zur GDP-Fucose nicht geeignet sind, entstehen selektiv, wenn kleinere Mengen an Säuren, wie z.B. Bortrifluorid, in den verwendeten Phosphaten vorhanden sind oder dem Reaktionsgemisch beigefügt werden. Werden erfindungsgemäß ausschließlich die $\beta$-Fucopyranosyl-phosphate gewünscht, so muß unbedingt säurefrei gearbeitet werden. Die Ausbeuten an dem $\beta$-Anomeren, bezogen auf das eingesetzte entsprechende $\alpha$-Trichloracetimidat, belaufen sich nach Aufreinigung erfindungsgemäß zwischen 85-95 %, vorzugsweise zwischen 85 und 90 %.

Vergleicht man die Ausbeuten an $\beta$-Fucopyranosyl-phosphat der erfindungsgemäßen Synthese, ausgehend im konkreten Fall von Tri-O-acetyl-L-fucose über die Imidatverbindung, mit den Ausbeuten der beiden bekannten chemischen Synthesen (Nunez et al., l. c.; Tsai et al., l. c.), so erhält man das gewünschte Produkt (als Salz) nach dem beschriebenen Verfahren mit durchschnittlich etwa 70 % Ausbeute, nach dem bekannten Verfahren von Nunez et al. mit etwa 50 % Ausbeute und nach dem Verfahren von Tsai et al. (ausgehend vom Orthoacetat der L-Fucose) mit nur etwa 20 % Ausbeute. Überdies weist das erfindungsgemäße Verfahren zur Herstellung von $\beta$-Fucopyranosyl-phosphaten, insbesondere im Vergleich zu dem Verfahren von Nunez et al., weniger und einfacherer Verfahrensschritte auf.

Das erfindungsgemäße Gesamtverfahren zeichnet sich durch eine hohe Stereoselektivität, eine geringe Anzahl von Verfahrensschritten, Einfachheit - so ist z.B. keine spezielle Trennung von $\alpha$- und $\beta$-Anomeren notwendig -, und sehr gute Ausbeuten aus. Aufgrund des neuartigen Aufreinigungsverfahren kann eine sehr reine GDP-Fucose, die auch für die Verwendung in den empfindlichsten biochemischen Prozesse geeignet ist, erhalten werden. Das Verfahren ist bestens geeignet, um im größeren technischen Maßstab eingesetzt zu werden.

Im folgenden sind die Abbildungen erläutert:

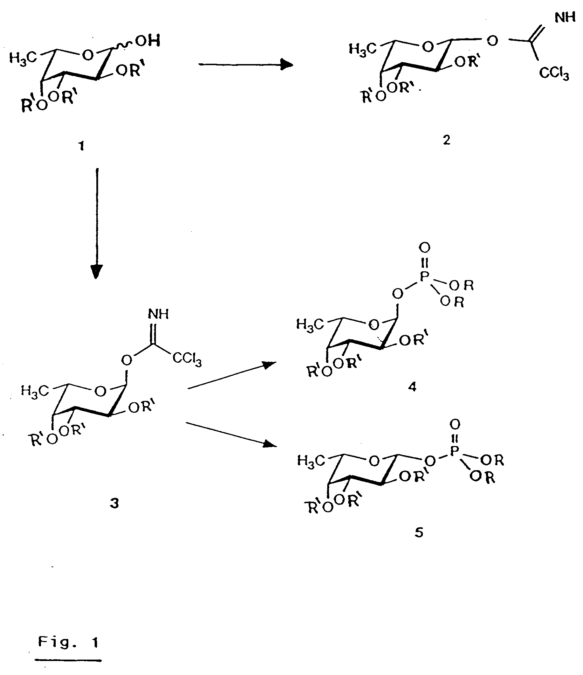

Fig. 1: Syntheseschema der Umsetzung von tri-substituierter L-Fucose zu den entsprechend geschützten $\alpha$- und $\beta$-L-Fucopyranosyl-phosphaten über die jeweiligen $\alpha$-Trichloracetimidate. 1: tri-substituierte L-Fucose; 2: $\beta$-Trichloracetimidat der geschützten L-Fucose; 3: $\alpha$-Trichloracetimidat der L-Fucose; 4: 2,3,4- geschütztes $\alpha$-L-Fucopyranosyl-phosphat; 5: 2,3,4- geschütztes $\beta$-L-Fucopyranosyl-phosphat; dabei bedeuten jeweils: R' = $R^1CO$-, $R^1$, $R^1$ = Alkyl ($C_1$-$C_4$), Benzyl, Phenyl, R = $R^1$.

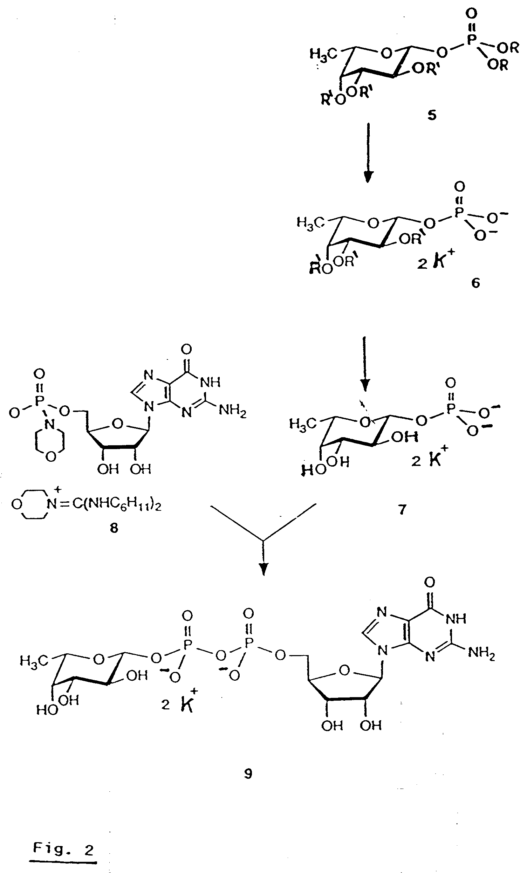

Fig. 2: Syntheseschema der Umsetzung von $\beta$-L-Fucopyranosyl-phosphat zu GDP-Fucose. 5: 2,3,4- geschütztes $\beta$-L-Fucopyranosyl-phosphat; 6: Salz des 2,3,4- geschützten $\beta$-L-Fucopyranosyl-phosphats; 7: Salz des ungeschützten $\beta$-L-Fucopyranosyl-phosphats; 8: Salz des (aktivierten) GMP-morpholidats; 9: GDP-Fucose (Salz); dabei bedeuten jeweils: R' = $R^1CO$-, $R^1$ = Alkyl ($C_1$-$C_4$), Benzyl, Phenyl, R = $R^1$, K = vorzugsw. organisches Kation.

Die im folgenden in Klammern stehenden Ziffern hinter einzelnen Verbindungen beziehen sich auf die Numerierung in den Abbildungen.

Vor- und nachstehend haben die Reste R, R' und $R^1$ die unten näher erläuterten Bedeutungen, sofern nicht ausdrücklich etwas anderes angegeben ist.

R und $R^1$ bedeuten $C_1$-$C_4$ - Alkyl, Benzyl oder Phenyl. Bedeuten R / $R^1$ Alkyl so sind dies im einzelnen Methyl, Ethyl, n-Propyl, Isopropyl, n-Butyl, Isobutyl, sec. und tert. Butyl; insbesondere bedeutet Alkyl Methyl.

Vorzugsweise bedeutet R bzw. $R^1$ aber Benzyl. R' bedeutet $R^1$CO- und $R^1$. Für die Synthese von GDP-Fucose ausgehend von den $\beta$-L-Fucopyranosyl-phosphaten bedeutet R' vorzugsweise $R^1$CO-. Innerhalb des Restes $R^1$CO- bedeutet $R^1$ vorzugsweise $CH_3$- oder $C_6H_4$-. R' bedeutet also vorzugsweise Acetyl oder Benzyl, insbesondere aber Acetyl. Bedeutet $R^1$ Phenyl oder Benzyl so können die aromatischen Ringe einfach oder mehrfach substituiert sein. Als Substituenten eignen sich F, Cl, Br, OH oder Methyl. Die aromatischen Ringe sind jedoch bevorzugt unsubstituiert. $K^+$ bedeutet ein anorganisches oder organisches Kation, vorzugsweise ein organisches Kation der Formel $NH(R^3)^+$, worin $R^3$ geradkettiges oder verzweigtes, vorzugsweise jedoch geradkettiges Alkyl mit 1 bis 10 C-Atomen bedeutet. Bevorzugt sind die kationischen Reste $NH(C_2H_5)_3^+$ und vor allem $NH(C_8H_{17})_3^+$.

Die Bedeutungen für einen bestimmten Rest innerhalb eines Moleküls können gleich oder verschiedenartig sein; vorzugsweise sind sie aber gleich.

Die einzelnen Schritte, die die Modifikation der Hydroxylgruppen der Fucose betreffen wie z.B. Veresterung, Veretherung, Halogenierung, Umesterung, Umetherung, Verseifung, Etherspaltung, Schutzgruppeneinführung und -abspaltung sowie Glycosylimidat-Bildung entsprechen, soweit nicht anderweitig beschrieben, den gängigen Standardmethoden der Kohlenhydratchemie. Eine umfassende Darstellung dieser Standardtechnologie ist beispielsweise zu finden in: Carbohydrate Chemistry (Edt. John F. Kennedy), Oxford Science Publication, Clarendon Press, 1988.

Das erfindungsgemäße Verfahren wird vorzugsweise wie folgt durchgeführt.

Um gezielt Änderungen am glycosidischen C-Atom bzw. am anomeren Kohlenstoff durchzuführen, bedarf es bekannterweise der Einführung von Schutzgruppen. Je nach gewünschter Reaktivität kommen hierfür bevorzugt Ester- oder Ethergruppierungen in Frage. Das erfindungsgemäße Verfahren bevorzugt hierbei als Schutzgruppen Acetyl bzw. Benzyl. Es können aber auch die oben näher spezifizierten Alternativen erfolgreich eingesetzt werden.

Synthese von tri-substituierter L-Fucose (1):

2,3,4-Tri-O-benzyl-L-fucopyranose (1) kann aus L-Fucose beispielsweise nach dem Verfahren von Wegmann et al. (Carbohydr. Res. 184 (1988), 254) oder von Dejter-Juszynski et al. (Carbohydr. Res. 18 (1971), 219) hergestellt werden. Erfindungsgemäß wird aber bevorzugt die triacetylierte L-Fucose synthetisiert, da sich diese für die weiteren Syntheseschritte besser eignet.

Zur Synthese von 2,3,4-Tri-O-acetyl-L-fucopyranose (1) wird erfindungsgemäß 1,2,3,4-Tetra-O-acetyl-$\alpha$-L-fucopyranose in einer Einstufen-Reaktion mit vorzugsweise Hydrazinacetat in Dimethylformamid umgesetzt. Zur gezielten Hydrolyse der anomeren Acetylgruppe eignet sich neben Hydrazinacetat auch noch Benzylamin, Kaliumhydroxid, Kaliumcyanid oder Bis(tributylzinn)oxid.

Das entstehende tri-acetylierte Produkt tritt in einem Anomeren-Verhältnis $\alpha$ : $\beta$ von durchschnittlich 3:1 auf. Für die weitere Reaktion und die Stereoselektivität des Gesamtverfahrens ist dieser Tatbestand jedoch unerheblich. Nach Aufreinigung über beispielsweise Kieselgel-Chromatographie erhält man das Anomerengemisch der tri-acetylierten L-Fucopyranose in einer Ausbeute von 75 bis 85 %. Das entsprechende Produkt wird in dem Verfahren des Standes der Technik (Nunez et al., l. c.) dagegen in einer aufwendigen Mehrstufenreaktion hergestellt und auch nicht weiter aufgereinigt. Das Ausgangsmaterial, die 1,2,3,4-Tetra-O-acetyl-$\alpha$-L-fucopyranose, ist, soweit es nicht im Handel erhältlich ist, gemäß der bekannten Verfahren nach Nunez et al. oder Prihar et al. (Biochemistry 12 (1973), 997) leicht zugänglich.

**Synthese der $\alpha$- und $\beta$- Trichloracetimidate von trisubstituierter L-Fucopyranose (2, 3):**

2,3,4-Tri-O-acetyl-L-fucopyranose (1) oder das entsprechend tri-benzylierte Derivat (1) werden nun mit Trichloracetonitril nach der bekannten Glycosylimidat-Methode (z.B. Schmidt et al., Angew. Chem. 92 (1980), 763) umgesetzt. Je nach Art der verwendeten Base läßt sich das Anomerenverhältnis steuern. Überraschend erhält man bei Einsatz von Natriumhydrid ausschließlich das für die weitere Synthese erforderliche O-(2,3,4-tri-O-acetyl/benzyl-$\alpha$-L-fucopyranosyl)- trichloracetimidat (3), also ausschließlich das $\alpha$-Anomer. Das gleiche Ergebnis erhält man bei Verwendung von Kaliumhydrid oder Natriumhydroxid. Setzt man dagegen andere Basen wie DBU (1,2-diazabicyclo[5.4.0] undec-7-en) oder Kaliumcarbonat ein, so erhält man ein Anomerengemisch von $\alpha$- und $\beta$- Trichloracetimidaten mit einem Verhältnis von etwa 7:1 bis 1:1. Das erfindungsgemäße Verfahren arbeitet demnach bevorzugt mit Basen, die wie Natriumh-

ydrid reagieren. Das $\alpha$-Trichloracetimidat wird vorzugsweise chromatographisch gereinigt und man erhält es danach in reiner Form mit einer Ausbeute von durchschnittlich 70 bis 80 %.

## Synthese von trisubstituierten L-Fucopyranosylphosphaten(4, 5)

Durch Umsetzung der wie oben beschriebenen dargestellten tri-substituierten $\alpha$-Trichloracetimidate der L-Fucose mit organischen Phosphaten der Formel $PO(OH)(OR)_2$, worin R $R^1$ bedeutet und $R^1$ die oben angegebene Bedeutung hat, erhält man in guten Ausbeuten die entsprechenden trisubstituierten L-Fucopyranosyl-phosphate, vorzugsweise die Dibenzyl-(2,3,4-tri-O-acetyl/benzyl-$\alpha$-L-fucopyranosyl)-phosphate (4) und die Dibenzyl-(2,3,4-tri-O-acetyl/benzyl-$\beta$-L-fucopyranosylphosphate (5). Als organisches Phosphat eignet sich also bevorzugt Dibenzylphosphat, zum einen weil es leicht erhältlich ist, zum anderen weil die Benzylgruppe besonders leicht selektiv abzuspalten ist.

Selbstverständlich sind auch die anderen oben aufgeführten Phosphate, wie z.B. Diphenylphosphat, geeignet. Entscheidend für die Eignung zur stereoselektiven Darstellung der $\alpha$- oder der $\beta$-konfigurierten Phosphate ist jedoch das Zugegensein bzw. das Fehlen von Säure während des Reaktionsverlaufes. Man erhält die reinen $\alpha$-Anomere (4), wenn man katalytische Mengen von Säuren, wie beispielsweise Bortrifluorid, dem Ansatz zugibt. Ist das eingesetzte Phosphat nicht zu rein, so kann sogar auf die Säurezugabe verzichtet werden. Arbeitet man hingegen absolut säurefrei, so erhält man selektiv das reine $\beta$-Anomere (5). Hierzu ist es in der Regel nötig, das eingesetzte Phosphat mindestens einmal frisch umzukristallisieren oder andere entsprechende Maßnahmen zu tätigen, die keine Säurekatalyse erlauben.

Die Ausbeuten belaufen sich nach Aufreinigung vorzugsweise zwischen 85 und 90 %, bezogen auf das eingesetzte $\alpha$-Trichloracetimidat der geschützten L-Fucose. Ohne spezielle Aufreinigung kann mit Ausbeuten zwischen 90 bis 98 % gerechnet werden. Die Ausbeuten sind von der Art der gewählten Ester-Schutzgruppe am Fucosering nicht entscheidend abhängig. Die Aufreinigung kann beispielsweise mittels Chromatographie an z.B. Kieselgel erfolgreich durchgeführt werden.

## Synthese von $\beta$-L-Fucopyranosyl-guanosin-5'-pyrophosphat (GDP-Fucose) (9).

Zur weiteren Synthese eignen sich bevorzugt die am Fucosering mit Estergruppen geschützten $\beta$-Fucopyranosyl-phosphaten, insbesondere die entsprechenden Acetyl- oder Benzoyl-Derivate.

Diese Schutzgruppen bleiben bei der folgenden Entfernung der Phosphat-Schutzgruppen, vorzugsweise durch Hydrogenolyse, erhalten. Bevorzugtes Substrat für die weitere Synthese ist demzufolge Dibenzyl- oder Diphenyl-(2,3,4-tri-O-acetyl-$\beta$-L-fucopyranosyl)-phosphat (5). Die Abspaltung vorzugsweise des Dibenzyl- bzw. Diphenylrestes kann beispielsweise mit Palladium und Wasserstoff nach Standardmethoden erfolgen. Aber auch andere Katalysatoren sind hierfür geeignet, ohne daß die Fucose-Schutzgruppen hydrolysiert werden, so z.B. Platin- und Nickelkatalysatoren. Die Hydrolyse kann auch mit Ammoniumformiat bewerkstelligt werden.

Das resultierende 2,3,4-Tri-O-acetyl-$\beta$-L-fucopyranosylphosphat (6) wird vorzugsweise als Salz, vorzugsweise als das Bis-triethylammonium-Salz, isoliert und gegebenenfalls chromatographisch in an sich bekannter Weise aufgereinigt. Aber auch die Isolierung als anderes Salz, z.B. als Barium- oder Bis-tri-n-butylammonium-Salz ist durchführbar. Der Reaktionsverlauf kann zweckmäßigerweise mittels Dünnschichtchromatographie verfolgt werden. Nun werden in einem weiteren Schritt die drei Schutzgruppen, vorzugsweise Acetyl, des Fucopyranosyl-Ringes in an sich bekannter Weise nach Standardmethoden entfernt und man erhält das entsprechende Salz des freien $\beta$-L-Fucopyranosyl-phosphats (7). Dieser letzte Schritt verläuft in der Regel quantitativ. In einer bevorzugten Ausführungsform des erfindungsgemäßen Verfahrens wird nun das so erhaltene $\beta$-Fucopyranosylphosphat in das entsprechende Tri-n-Octylammonium-Salz übergeführt. Diese erweist sich in dem anschließenden letzten Syntheseschritt überraschenderweise als besonders günstig, wahrscheinlich aufgrund seiner günstigen Löslichkeitseigenschaften. Auch in diesem Sinne vergleichbare andere Ammoniumsalze der Formel $NH(R^3)^+$ $X^-$, worin $R^3$ die oben definierten Bedeutungen haben, insbesondere worin $R^3$ höherkettiges Alkyl bedeutet, sind vorzüglich geeignet. Die Umsetzung des vorzugsweise verwendeten Bis-tri-n-octylammonium-($\beta$-L-fucopyranosyl)-phosphats zum Endprodukt, GDP-Fucose, der erfindungsgemäßen Synthese wird mit Guanosin-Monophosphat durchgeführt. Dabei ist es unerläßlich, wie in der Literatur beschrieben, daß GMP in einer aktivierten Form eingesetzt wird, um gute Umsetzungsraten zu erzielen. Als besonders geeignet hat sich die Kondensation mit GMP-5'-phosphormorpholidat erwiesen. Aber auch anderweitig bekanntermaßen aktiviertes GMP, wie beispielsweise bei Nunez et al. beschrieben, ist einsetzbar. In einer bevorzugten Ausführungsform des Verfahrens wird 4-Morpholin-N,N'-dicyclohexyl-carboxamidinium-guanosin-5'-monophosphormorpholidat (8) eingesetzt. Es entsteht das Bis-tri-n-octylammonium-Salz der L-GDP-Fucose (9) mit einer durchschnittlichen Ausbeute von etwa 70 %. Zur weiteren Reinigung wird die Verbindung wiederum in das für diese Zwecke günstigere Bis-triethylammonium-Salz übergeführt. Erfindungsgemäß erfolgt nun eine Aufreinigung mittels HPLC, vorzugsweise an Reverse Phase Materialien. Entscheidend für die guten Endausbeuten an hochreiner GDP-Fucose und im Sinne einer einfachen Verfahrenstechnik ist weiterhin, daß das Syntheseendprodukt mit einem leicht verdampfbaren Puffersystem von einer vorzugsweise präparativen Säure eluiert wird. Als derartig einsetzbare Eluenten eignet sich vor allem Triethylammoniumhydrogencarbonat. Alternativ kommen ferner in Frage Ammoniumhydrogencarbonat oder Ammoniumformiat.

Die beschriebene Aufreinigungsweise hat den Vorteil, daß sehr einfach sehr reine GDP-Fucose erhalten werden kann. Eine Entsalzung, die erfahrungsmäßig stets Ausbeuteverluste mit sich bringt, kann somit entfallen.

Die folgenden Beispiele beschreiben die Erfindung in konkretisierter Form.

Generell wurden hierbei folgende Chromatographiesysteme verwendet:

Dünnschichtchromatographie: DC Plastikfolien Kieselgel 60 $F_{254}$ (E. Merck, Darmstadt, FRG), Detektion im UV-Licht bei 254 nm, bzw. mittels Besprühen mit 10 %iger $H_2SO_4$ und Erwärmen auf 110 °C.

Säulenchromatographie: Kieselgel 60, Korngröße 0.063 - 0.200 nm (E. Merck, Darmstadt, FRG).

HPLC analytisch: LiChrospher RP-18, 125 x 4 mm, 5 $\mu$m (E. Merck, Darmstadt, FRG), Puffer: 0.05 M Triethylammoniumhydrogencarbonat (pH 7.1) 3.5 %ig an Acetonitril, 0.5 ml/min. $R_f$ = 3.24 min (GMP), 3.85 min (GDP-Fucose), 12.7 min (GMP-Morpholidat).

HPLC präparativ: LiChrosorb RP-18, 250 x 10 mm, 7 $\mu$m (E. Merck, Darmstadt, FRG) Puffer wie oben.

Die synthetisierten Zwischen- Endprodukte wurden mittels $^1$H-NMR und $^{13}$C-NMR identifiziert und ihr Reinheitsgrad bestimmt.

**Beispiel 1:**

Eine Lösung von 2,3,4-Tri-O-benzyl-L-fucopyranose (hergestellt z.B. nach Wegmann et al., l. c.) (0.50 g, 0.86 mmol) in 20 ml trockenem Dichlormethan und handelsüblichem, nicht weiter aufgereinigten Dibenzylphosphat (0.24 g, 0.86 mmol) wird unter Stickstoffatmosphäre bei Raumtemperatur etwa 2 h gerührt. Bei sehr reinem Dibenzylphosphat wird eine katalytische Menge von Bortrifluorid dem Ansatz beigefügt. Die Lösung wird anschließend eingeengt und chromatographisch aufgereinigt (Toluol/Aceton 9:1). Man erhält Dibenzyl-(2,3,4-tri-O-benzyl-$\alpha$-L-fucopyranosyl)-phosphat (4) in einer Ausbeute von 90 % als farbloses Öl (0.54 g). DC (Toluol/Aceton 7:1) ergibt einen $R_f$-Wert von 0.60, $[\alpha]_{578}$ = -68.2 (c = 1, Chloroform).

**Beispiel 2:**

Eine Lösung von 2,3,4-Tri-O-benzyl-L-fucopyranose (0.50 g, 0.86 mmol) in 20 ml trockenem Dichlormethan und frisch umkristallisierten Dibenzylphosphat (0.24 g, 0.86 mmol) wird unter Stickstoffatmosphäre bei Raumtemperatur etwa 1 h gerührt. Die Lösung wird anschließend eingeengt und chromatographisch aufgereinigt (Toluol/Aceton 7:1). Man erhält Dibenzyl-(2,3,4-tri-O-benzyl-$\beta$-L-fucopyranosyl)-phosphat (5) in einer Ausbeute von 95 % als farbloses Öl (0.57 g). DC (Toluol/Aceton 7:1 ergibt einen $R_f$-Wert von 0.45, $[\alpha]_{578}$ = -9.7 (c = 1, Chloroform).

**Beispiel 3:**

Eine Lösung von 1,2,3,4-Tetra-O-acetyl-$\alpha$-L-fucopyranose (6.00 g, 18 mmol), hergestellt z.B. nach Nunez et al.; (l. c.) und Hydrazinacetat (1.99 g, 21.6 mmol) in 20 ml trockenem Dimethylformamid wird bei 50 °C etwa 4.5 h lang gerührt. Nach Zugabe von Essigsäureethylester wird die Mischung etwa zweimal mit wäßriger Natriumchlorid-Lösung extrahiert und der eingeengte organische Extrakt chromatographiert (Petrolether (40-60°)-/Essigsäureethylester 1:1). Man erhält 2,3,4-Tri-O-acetyl-L-fucopyranose (1) in einem Anomerenverhältnis von $\alpha$ : $\beta$ von 3:1 mit einer Ausbeute von 80 %. DC-Analyse (Petrolether (40-60°)-/Essigsäureethylester 1:1) ergibt $R_f$ = 0.47.

**Beispiel 4:**

(a) Zu einer Mischung von 2,3,4-Tri-O-acetyl-L-Fucopyranose (Anomerengemisch) (2.00 g, 6.89 mmol) und Trichloracetonitril (7.0 ml, 70 mmol) in 20 ml trockenem Dichlormethan wird unter Stickstoff und bei Raumtemperatur Natriumhydrid (0.25 g, 10.9 mmol) zugesetzt. Man rührt ca. 12 h lang, filtert dann die Mischung durch Kieselgur und engt sie ein. Nach chromatographischer Aufreinigung (Petrolether/Essigsäureethylester 3:1) erhält man O-(2,3,4-Tri-O-acetyl-$\alpha$-L-fucopyranosyl)-trichloracetimidat (3) in einer Ausbeute von 71 % (2.13 g).

b) Eine Mischung von 2,3,4-Tri-O-acetyl-L-Fucopyranose (Anomerengemisch) (0.50 g, 1.72 mmol), Lithiumchlorid (73 mg, 1.72 mmol), 1,8-Diazabicyclo-[5.4.0]-undec-7-en (0.20 ml, 1.34 mmol) und Trichloracetonitril (2.0 ml, 20 mmol) in 20 ml trockenem Acetonitril wird unter Stickstoff und bei Raumtemperatur etwa 12 h gerührt. Nach chromatographischer Aufreinigung (Petrolether/Diethylether 2:5) erhält man ein Gemisch aus O-(2,3,4-Tri-O-acetyl-α-L-fucopyranosyl)-trichloracetimidat (3) und O-(2,3,4-Tri-O-acetyl-β-L-fucopyranosyl)-trichloracetimidat (2) in einer Ausbeute von 92 % (α : β = 6.5:1).

c) Eine Mischung von 2,3,4-Tri-O-acetyl-L-Fucopyranose (Anomerengemisch) (0.85 g, 2.93 mmol), Trichloracetonitril (3 ml, 30 mmol) und Kaliumcarbonat (2.73 g, 19`7 mmol) in trockenem Dichlormethan (12 ml) wird unter Stickstoff bei Raumtemperatur etwa 4 h lang gerührt. Nach Filtration durch Kieselgur wird das Lösungsmittel abgezogen. Nach chromatographischer Aufreinigung (Petrolether/Diethylether 2:5) erhält man ein Gemisch aus O-(2,3,4-Tri-O-acetyl-α-L-fucopyranosyl)-trichloracetimidat (3) und O-(2,3,4-Tri-O-acetyl-β-L-fucopyranosyl)-trichloracetimidat (2) in einer Ausbeute von 76 % (α : β = 1:1.3).

α-Anomer: $[\alpha]_D$ = -116.0 (c = 1, Chloroform), $R_f$ = 0.54 (Petrolether/Essigsäureethylester 2:1), Fp. 107-109 °C.

β-Anomer: $[\alpha]_D$ = -24.5 (c = 1, Chloroform), $R_f$ = 0.34 (Petrolether/Essigsäureethylester 2:1), Fp. 60-63 °C.

**Beispiel 5:**

Eine Lösung von O-(2,3,4-Tri-O-acetyl-α-L-fucopyranosyl)-trichloracetimidat (0.20 g, 0.46 mmol) in trockenem Dichlormethan (7 ml) und handelsüblichen, nicht weiter aufgereinigten Dibenzylphosphat (0.13 g, 0.47 mmol) wird unter Stickstoff bei Raumtemperatur etwa 2.5 h lang gerührt. Bei sehr reinem Dibenzylphosphat wird eine katalytische Menge von Bortrifluorid dem Ansatz beigefügt. Die Lösung wird anschließend eingeengt und chromatographisch aufgereinigt (Toluol/Aceton 7:1) und rechromatographiert (Chloroform/Diethylether 20:1). Man erhält Dibenzyl-(2,3,4-tri-O-acetyl-α-L-fucopyranosyl)-phosphat (4) in einer Ausbeute von 91 % als farbloses Öl (0.23 g). DC (Toluol/Aceton/Triethylamin 84:15:1) ergibt einen $R_f$-Wert von 0.38, $[\alpha]_D$ = -83.7 (c = 1, Chloroform).

**Beispiel 6:**

Eine Lösung von O-(2,3,4-Tri-O-acetyl-α-L-fucopyranosyl)-trichloracetimidat (0.50 g, 1.15 mmol) in trockenem Dichlormethan (25 ml) und handelsüblichen, nicht weiter aufgereinigten Diphenylphosphat (0.29 g, 1.16 mmol) wird unter Stickstoff bei Raumtemperatur etwa 6.5 h lang gerührt. Bei sehr reinem Dibenzylphosphat wird eine katalytische Menge von Bortrifluorid dem Ansatz beigefügt. Die Lösung wird anschließend eingeengt und chromatographisch aufgereinigt (Petrolether/Diethylether 1:5). Man erhält Diphenyl-(2,3,4-tri-O-acetyl-α-L-fucopyranosyl)-phosphat (4) in einer Ausbeute von 63 % als farbloses Öl (0.38 g). DC (Petrolether/Diethylether 1:5) ergibt einen $R_f$-Wert von 0.41.

**Beispiel 7:**

Eine Lösung von O-(2,3,4-Tri-O-acetyl-α-L-fucopyranosyl)-trichloracetimidat (0.30 g, 0.69 mmol) in trockenem Dichlormethan (12 ml) und handelsüblichen, aber frisch umkristallisierten Dibenzylphosphat (0.19 g, 0.68 mmol) wird unter Stickstoff bei Raumtemperatur etwa 1 h lang gerührt. Die Lösung wird anschließend eingeengt und chromatographiert: (Toluol/Aceton 7:1) und rechromatographiert (Petrolether/Diethylether 1:5). Man erhält Dibenzyl-(2,3,4-tri-O-acetyl-β-L-fucopyranosyl)-phosphat (5) in einer Ausbeute von 86 % als farbloses Öl (0.36 g). DC (Toluol/Aceton 7:1) ergibt einen $R_f$-Wert von 0.30, $[\alpha]_D$ = +0.5 (c = 1, Chloroform).

**Beispiel 8:**

Eine Mischung von Dibenzyl-(2,3,4-tri-O-acetyl-β-L-fucopyranosyl)-phosphat (5) (72 mg, 0.13 mmol) und Palladium (4 mg) in 10 ml trockenem Tetrahydrofuran/Essigsäureethylester (1:1) wird und $H_2$-Atmosphäre geschüttelt. Das Ende der Reaktion wird mittels DC ($R_f$ = 0.19, Chloroform/Methanol 60:40) bestimmt. Die Mischung wird gefiltert, eingeengt und chromatographiert (Chloroform/Methanol/Triethylamin 60:39:1). Nach Lyophilisierung in Dioxan erhält man Bis-triethylammonium-(2,3,4-tri-O-acetyl-β-L-fucopyranosyl)-phosphat (6) in einer Ausbeute von 90 % (68 mg) als amorphes Pulver.

**Beispiel 9:**

Zu einer Lösung von Bis-triethylammonium-(2,3,4-tri-O-acetyl-β-L-fucopyranosyl)-phosphat (80.0 mg, 0.135 mmol) in 3 ml Methanol/Wasser-Gemisch fügt man 0.5 ml Triethylamin. Nach 18 h wird die Mischung eingeengt und aus Wasser lyophilisiert. Man erhält Bis-triethylammonium-(β-L-fucopyranosyl)-phosphat (7) in einer Ausbeute von 100 % (73.8 mg) als hygroskopisches weißes Pulver, das nicht weiter aufgereinigt wird.

**Beispiel 10:**

Zu einer Lösung von Bis-triethylammonium-(β-L-fucopyranosyl)-phosphat (73.8 mg, 0.135 mmol) in trockenem Pyridin (5 ml) wird Tri-n-octylamin (0.117 ml, 0.135 mmol) zugesetzt. Die Mischung wird eingeengt und das resultierende Tri-n-octylammonium-Salz wird durch wiederholtes Eindampfen mit Pyridin bei $10^{-2}$ Torr getrocknet. Nach Zugabe von 4-Morpholin-N,N'-dicyclohexyl-carboxamidinium-guanosin-5'-monophosphormorpholidat (8) (109 mg, 0.15 mmol) in trockenem Pyridin/Dimethylformamid (1:1) wird die Reaktion mittels analytischer HPLC verfolgt. Nach etwa 5 Tagen wird die Mischung zur Trockene eingeengt. Der Rückstand wird in Triethylammoniumhydrogencarbonat-Puffer (pH 7.4, 0.05 M) aufgenommen, Tri-n-octylamin durch Extraktion mit Ether entfernt und abschließend mittels präparativer HPLC ($t_R$ = 9.4 min) aufgereinigt und der als Eluent dienende Puffer abgedampft. Man erhält sehr reines Bis-triethylammonium-($\beta$-L-fucopyranosyl)-guanosin-5'-pyrophosphat (9) in einer Ausbeute von 25 % (23.3 mg) als amorphes Pulver.

**Patentansprüche**

1. Verfahren zur stereoselektiven Herstellung von $\beta$-L-Fucopyranosyl-phosphaten. dadurch gekennzeichnet, daß man an den Positionen 2, 3 und 4 geschützte L-Fucose in das entsprechende O-($\alpha$-L-Fucopyranosyl)-trichloracetimidat überführt, dieses mit einem absolut säurefreiem organischem Phosphat der Formel PO-(OH)(OR)$_2$, worin R $C_1$-$C_4$-Alkyl, Phenyl oder Benzyl bedeutet, umsetzt, den Rest R der Phosphatgruppe und die Schutzgruppen am Fucopyranosylring abspaltet und das $\beta$-L-Fucopyranosyl-phosphat als Salz isoliert.

2. Verfahren nach Anspruch 1, dadurch gekennzeichnet, daß als Schutzgruppen Acetyl oder Benzyl eingesetzt werden.

3. Verfahren nach Anspruch 1 oder 2, dadurch gekennzeichnet, daß die geschützte L-Fucose mit Natriumhydrid selektiv zum entsprechenden $\alpha$-Trichloracetimidat umgesetzt wird.

4. Verfahren nach einem der Ansprüche 1 bis 3, dadurch gekennzeichnet, daß man 2,3,4-Tri-O-acetyl-L-fucose aus der tetra-acetylierten L-Fucose in einer Einstufen-Reaktion herstellt.

5. Verfahren nach Anspruch 4, dadurch gekennzeichnet, daß man die anomere Acetyl-Gruppe der Tetra-O-acetyl-L-fucose mit Hydrazinacetat abspaltet.

6. Verfahren zur stereoselektiven Herstellung von sehr reiner Guanosin-diphosphat-fucose (GDP-Fucose), dadurch gekennzeichnet, daß man $\beta$-L-Fucopyranosyl-phosphat in ein leicht lösliches Salz überführt, dieses mit aktiviertem Guanosin-monophosphat (GMP) umsetzt und die gebildete GDP-Fucose mittels präparativer HPLC mit einem leicht verdampfbaren Puffersystem als Eluent aufreinigt.

7. Verfahren nach Anspruch 6, dadurch gekennzeichnet, daß man $\beta$-L-Fucopyranosyl-phosphat in das Tri-n-octylammonium-Salz überführt.

8. Verfahren nach Anspruch 6 oder 7, dadurch gekennzeichnet, daß man als aktiviertes GMP GMP-Morpholidat einsetzt.

9. Verfahren nach einem der Ansprüche 6 bis 8, dadurch gekennzeichnet, daß als Eluent für die präparative Aufreinigung Triethylammoniumhydrogencarbonat eingesetzt wird.

10. Verfahren zur Herstellung von GDP-Fucose nach einem der Ansprüche 6 bis 9, dadurch gekennzeichnet, daß das eingesetzte $\beta$-L-Fucopyranosyl-phosphat nach einem der Ansprüche 1 bis 5 hergestellt wird.

Fig. 1

Fig. 2